# EUROPÄISCHE PATENTANMELDUNG

(11) **EP 1 655 018 A1**
(43) Veröffentlichungstag der Anmeldung: **10.05.2006**
(21) Anmeldenummer: 05014862.6
(22) Anmeldetag: 08.07.2005
(51) Int. Cl.: A61K 8/34, A61K 8/64, A61Q 11/00

(54) **Mittel für die Reinigung und Pflege des Mundraums und der Zähne mit entzündungshemmender Wirkung**

(30) Priorität: 08.09.2004 DE 102004043802
(71) Anmelder: Henkel Kommanditgesellschaft auf Aktien, 40589 Düsseldorf (DE)
(72) Erfinder: Leinen, Hans-Theo, 40669 Erkrath (DE); Wülknitz, Peter, 42799 Leichlingen (DE)

(57) **Zusammenfassung**

Mund- und Zahnpflege- und -reinigungsmittel, die Feuchthaltemittel und mindestens ein Protein aus Leguminosensamen enthalten, besitzen entzündungshemmende Wirkung und fördern die Reparatur von bereits geschädigtem Zahnfleisch.

## Beschreibung

Die Erfindung betrifft Zubereitungen zur Mund- und Zahnpflege- und -reinigung, die aufgrund einer speziellen Wirkstoffkombination Entzündungen des Zahnfleisches (Gingivitis) entgegenwirken.

Die Mundhöhle des Menschen wird von einer großen Anzahl kommensalisch lebender Bakterien, der residenten Mikroflora, besiedelt, welche an die speziellen Bedingungen im Mundraum angepaßt sind. Die Mikroflora der Mundhöhle schützt vor der Kolonisierung durch pathogene Keime und ist für die Geruchsbildung, also den Mundgeruch, verantwortlich. Die Bakterien nutzen das Nahrungsangebot in der Mundhöhle und bilden beim Abbau der Nährstoffe riechende Substanzen wie beispielsweise kurzkettige Fettsäuren. Die mikrobielle Besiedlung und Ablagerung von Stoffwechselprodukten auf den Zähnen begünstigt die Plaque-Bildung. Die mikrobiellen Abbauprodukte aus Kohlenhydraten aus der Nahrung führen zu einer Reduktion des pH-Wertes und unterstützen die Karies-Bildung. Infolge der Säurebildung der Plaque-Bakterien kommt es zu einer Mikroentkalkung der Zähne. Die Bakterien sollten durch regelmäßige Plaqueentfernung und Beschränkung der häufigen Zuckeraufnahme bzw. Unterstützung der Wiederverkalkung durch häufige Fluoridierung geschützt werden.

Zahnpflegemittel dienen in erster Linie der Reinigung der Zahnoberfläche von Speiseresten, Verfärbungen und fest anhaftenden bakteriellen Zahnbelägen. Darüber hinaus wird versucht, durch spezielle Zusätze, z.B. durch Fluorverbindungen oder antimikrobielle Stoffe den Erkrankungen der Zähne und des Zahnfleisches wie z.B. Karies, Gingivitis oder Parodontose entgegenzuwirken.

Eine besonders hartnäckige Erkrankung, die - wenn sie nicht erfolgreich bekämpft wird - zur Lockerung und zum Verlust von Zähnen führen kann, ist die Parodontitis, die im Anfangsstadium durch Entzündung und Blutung des Zahnfleisches (Gingivitis) in Erscheinung tritt. Sie wird durch Bakterien verursacht, die in den Zahntaschen siedeln und durch mechanische Reinigung der Zähne mit der Zahnbürste nur schwer zu bekämpfen sind.

Man hat versucht, durch Zusatz von antibakteriellen Stoffen in Zahnpasten und Mundwässern Zahnfleischentzündungen zu bekämpfen, ein durchschlagender Erfolg ist aber in den meisten Fällen damit nicht zu erzielen. Als besonders wirksam haben sich zwar bestimmte nicht-kationische bakterizide Stoffe, insbesondere aus der Gruppe der chlorierten Diphenylether erwiesen, aber auch diese Stoffe sind allein nicht in der Lage, die hartnäckigen Zahnfleischentzündungen wirksam zu bekämpfen.

Der vorliegenden Erfindung lag die Aufgabe zugrunde, Mund- und Zahnpflege- und - reinigungsmittel mit verbesserter entzündungshemmender Wirkung bereitzustellen. Darüber hinaus sollten die bereitzustellenden Mittel eine gute Verträglichkeit aufweisen und nach Möglichkeit weitere Vorteile bei der Anwendung bewirken können.

Es wurde nun gefunden, daß sich Mund- und Zahnpflege- und -reinigungsmittel mit entzündungshemmender Wirkung bereitstellen lassen, wenn man bestimmte Proteine in diese Mittel einarbeitet. Darüber hinaus wurde gefunden, daß die Mund- und Zahnpflege- und -reinigungsmittel, die diese Proteine enthalten, die Reparatur von bereits geschädigtem Zahnfleisch fördern.

Ein erster Gegenstand der vorliegenden Erfindung sind Mund- und Zahnpflege- und - reinigungsmittel, enthaltend Feuchthaltemittel und mindestens ein Protein aus Leguminosensamen.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahn-reinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Die erfindungsgemäßen Mittel enthalten als ersten wesentlichen Bestandteil mindestens ein Protein aus Leguminosensamen. Als Leguminosensamen zur Gewinnung der Proteine, die in den erfindungsgemäßen Mitteln enthalten sind, eignen sich Samen von Bohnenarten wie Phaseolus angularis, Phaseolus lunatus, Phaseolus aureus, Phaseolus vulgaris, Phaseolus coccineus, Phaseolus limensis , Erbsenarten wie Lathyrus odoratus, die Soyabohnen Glycine max und Glycine hispida, die Erdnuss Arachis hypogaea sowie die Samen tropischer Leguminosen der Gattungen Cajanus, Dolichus, Vigna und Vicia.

Die Proteinfraktionen, die in den erfindungsgemäßen Mitteln enthalten sind, können aus den genannten Leguminosensamen gewonnen werden, indem man die getrockneten Samen mahlt, das erhaltene Mehl mit einem organischen Lösungsmittel oder einem Lösungsmittelgemisch extrahiert, trocknet und das derart entfettete Mehl mit Wasser oder einer wässrigen Elektrolytlösung bei einem pH von 2 bis 10, vorzugsweise bei pH 5 bis 6 extrahiert, den Extrakt auf pH 5 bis 7 stellt, im Vakuum einengt, das Konzentrat unter Zusatz eines Filterhilfsmittels wie zum Beispiel Kieselgur klar filtriert oder zentrifugiert, die Proteine daraus entweder durch Salzfällung, beispielsweise mit Ammoniumsulfat bei 30 bis 80% Sättigung, oder durch Fällung mit einem organischen, wassermischbaren Lösungsmittel wie zum Beispiel Ethanol in einer Konzentration von 60 bis 90% abscheidet, durch Filtration oder Zentrifugation sammelt und schließlich entweder direkt im Vacuum trocknet oder vorerst Salze durch Dialyse, Gelfiltration oder Ultrafiltration entfernt und dann lyophilisiert.

Erfindungsgemäß besonders bevorzugte Mund- und Zahnpflege- und -reinigungsmittel sind dadurch gekennzeichnet, daß sie ein Protein enthalten, das gekennzeichnet ist durch
- a) seine Isolierung aus Leguminosensamen,
- b) mindestens eine Bande in der Polyacrylamidgelelektrophorese mit Natriumdodecylsulfat,
- c) Molekularmassen von 3000 bis 30'000 g/mol,
- d) Gehalt an Gesamtstickstoff von 14% bis 20% und Aminostickstoff von 1% bis 2% bezogen auf den Proteingehalt,
- e) Löslichkeit in Wasser und wässrigen Elektrolytlösungen und Unlöslichkeit in Ethanol und Aceton,
- f) starke Ausfällung in wässriger Lösung nach Zusatz von Trichloressigsäure, Sulfosalicylsäure, Pikrinsäure oder Benzethoniumchlorid,
- g) Hemmung von PMN-Elastase und Fibroblastenelastase.

Erfindungsgemäße Mittel enthalten mindestens ein Protein aus Legmuniosensamen [Merkmal a)]. Die vorstehend beschriebenen erfindungsgemäß bevorzugten Mittel sind darüber hinaus dadurch gekennzeichnet, daß das Protein bzw. die Proteinfraktion in der Polyacrylamidgelelektrophorese in Anwesenheit von Natriumdodecylsulfat (Phastsystem, Pharmacia Biosystems, Uppsala, S) mindestens eine Bande aufweist, deren elektrophoretische Mobilität auf relative Molekularmassen von 3000 bis 30 000 g/mol schliessen lässt [Merkmale b) und c)]. Darüber hinaus ist ein Gehalt an Gesamtstickstoff von 14 bis 20% und an Aminostickstoff von 1 bis 2%, jeweils bezogen auf den Proteingehalt, bevorzugt [Merkmal d)], und das Protein bzw. die Proteinfraktion ist vorzugsweise in Wasser und wässrigen Elektrolytlösungen löslich, in Ethanol und Aceton unlöslich und zeigt in wässriger Lösung nach Zusatz von Trichloressigsäure, Sulfosalicylsäure, Pikrinsäure oder Benzethoniumchlorid eine starke Ausfällung [Merkamle e) und f)]. Ferner hemmt die Proteinfraktion Proteinasen und zeigt z.B., gemessen an Trypsin, einen I₅₀-Wert von unter 10 µg (bezogen auf die Trockensubstanz) pro ml Testgemisch, gemessen an PMN-Elastase, einen I₅₀-Wert von unter 100 µg (bezogen auf die Trockensubstanz) pro ml, gemessen an Tryptase, einen I₅₀-Wert von unter 200 mg (bezogen auf die Trockensubstanz) pro ml Testgemisch und gemessen an Fibroblastenelastase, einen I₅₀-Wert von unter 350 mg (bezogen auf die Trockensubstanz) pro ml Testgemisch.

Als bevorzugte Quelle für die Proteine aus Leguminosensamen haben sich die Samen von Sojabohnen herausgestellt. Demnach sind erfindungsgemäße Mund- und Zahnpflege- und ―reinigungsmittel bevorzugt, die mindestens ein Protein aus Sojasamen enthalten.

Das bzw. die Protein(e) aus Leguminosensamen können in den erfindungsgemäßen Mitteln in Mengen von bis zu 10 Gew.% enthalten sein, wobei die entzündungshemmende und zahnfleischreparierende Wirkung bereits bei deutlich niedrigeren Konzentration eintritt. Besonders bevorzugte erfindungsgemäße Mund- und Zahnpflege- und ―reinigungsmittel sind dadurch gekennzeichnet, daß sie das/die Protein(e) aus Leguminosensamen in Mengen von 0,0001 bis 5 Gew.%, vorzugsweise von 0,001 bis 2,5 Gew.% und insbesondere von 0,01 bis 1 Gew.%, jeweils bezogen auf das gesamte Mittel, enthalten.

Als zweiten wesentlichen Bestandteil enthalten die erfindungsgemäßen Mund- und Zahnpflege- und ―reinigungsmittel mindestens ein Feuchthaltemittel. Hier sind erfindungsgemäße Mund- und Zahnpflege- und ―reinigungsmittel bevorzugt, die ein Feuchthaltemittel aus der Gruppe der Alkohole mit mindestens 2 OH-Gruppen, vorzugsweise Glycerin, Sorbit, Mannit, Xylitol, Polyethylenglycol, Polypropylenglycol und deren Mischungen, enthalten.

Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 1, 2, 3, 4, 5, 6, 7, 8, 9, 10, 11, 12, 13, 14, 15, 16, 17, 18, 19, 20. Auch 1,3-Diole wie H-(CH₂)ₙ₋CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen- und Polypropylenglycole.

Unter den Polymhydroxyverbindungen mit 3 OH-Gruppen hat das Glycerin eine herausragende Bedeutung.

Als bevorzugte Feuchthaltemittel können z. B. Glycerin, Sorbit, Xylit, Propylenglycole, Polyethylenglycole, insbesondere solche mit mittleren Molekulargewichten von 200―800 eingesetzt werden. Bevorzugt enthalten die erfindungsgemäßen Zahnpflegemittel als Feuchthaltemittel ein Gemisch aus Glycerin, Sorbit und Polyethylenglycol im Gewichtsverhältnis 10 : (8 ― 12) : (0,1 ― 1).

Das bzw. die Feuchthaltemittel können in den erfindungsgemäßen Mitteln in variierenden Mengen eingesetzt werden. Während in Abhängigkeit von den übrigen optionalen Inhaltsstoffen Mengen von wenigen % (beispielsweise 1, 2, 3, 4, 5, 6, 7, 8, 9, 10 Gew.%, jeweils bezogen auf das gesamte Mittel) enthalten sein können, existieren auch Formulierungen, die deutlich höhere Feuchthaltemittel-Gehalte, beispielsweise von 50, 55, 60, 65, 70, 75 Gew.%, jeweils bezogen auf das gesamte Mittel, aufweisen. Üblich und bevorzugt sind erfindungsgemäße Mittel, die zwischen 10 und 75 Gew.% Feuchthaltemittel enthalten. Gehalte zwischen 15 und 70, vorzugsweise zwischen 20 und 60 Gew.%, jeweils bezogen auf das gesamte Mittel, können bevorzugt sein.

Die erfindungsgemäßen Mund- und Zahnpflege- und ―reinigungsmittel können zusätzlich zu den vorstehend genannten wesentlichen Inhaltsstoffen weitere Inhaltsstoffe von Mundreinigungsmitteln, Mundpflegemitteln, Zahnreinigungsmitteln und/oder Zahnpflegemitteln enthalten. Die bevorzugten weiteren Inhaltsstoffe werden nachstehend beschrieben.

Die erfindungsgemäßen Mund- und Zahnpflege- und reinigungsmittel, insbesondere die Zahnpasten, können z. B. auch antimikrobielle Stoffe als Konservierungsmittel oder als Anti- Plaque-Wirkstoffe enthalten. Derartige Stoffe können z. B. ausgewählt sein aus p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol usw.. Erfindungsgemäß bevorzugte Mund- und Zahnpflege- und ― reinigungsmittel sind dadurch gekennzeichnet, daß sie zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel, enthalten.

Eine weitere bevorzugte Gruppe von Inhaltsstoffen, die in den erfindungsgemäßen Mitteln enthalten sein kann, sind die Antikaries-Wirkstoffe. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und ―reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Die Mund- und Zahnpflege- und reinigungsmittel, insbesondere die Zahnpasten, können z. B. auch Substanzen enthalten, die gegen Zahnstein wirksam sind. Derartige Substanzen können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Die erfindungsgemäßen Mittel, insbesondere die Zahnpasten oder flüssigen Zahncremes enthalten vorzugsweise ein oder mehrere Poliermittel, üblicherweise in einer Menge von 5 bis 50 Gew.-%.

Als Poliermittel eignen sich prinzipiell alle für Zahnpasten bekannten Reibkörper, insbesondere solche, die keine Calciumionen enthalten. Bevorzugt geeignete Poliermittelkomponenten sind daher Kieselsäuren, Aluminiumhydroxid, Aluminiumoxid, Natriumaluminiumsilikate, organische Polymere oder Gemische solcher Reibkörper.

Calciumhaltige Polierkomponenten wie z.B. Kreide, Calciumpyrophosphat, Dicalciumphosphat-dihydrat können aber in Mengen bis zu 5 Gew.-% enthalten sein.

Der Gesamtgehalt an Poliermitteln liegt vorzugsweise im Bereich von 5 ― 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m2/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 ―14 µm bei einer spezifischen Oberfläche von 40 ― 75 m2/g (nach BET) aus und eignen sich besonders gut für flüssige Zahncremes. Diese sollten eine Viskosität (25°C, Scherrate D = 10 s-1) von 10 ― 100 Pa.s aufweisen.

Zahnpasten, die eine deutlich höhere Viskosität von mehr als 100 Pa.s (25° C, D = 10 s-1) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 ― 3 µm. Solchen Zahnpasten setzt man daher bevorzugt neben den genannten Fällungskieselsäuren noch feinteiligere, sogenannte Verdickungskieselsäuren mit einer BET-Oberfläche von 150 ―250 m2/g zu, z.B. die Handelsprodukte Sipernat 22 LS oder Sipernat 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 ― 5 Gew.-% enthalten sein. Ein solches geeignetes Aluminiumoxid ist unter der Handelsbezeichnung "Poliertonerde P 10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und ―reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ 2H₂O), Natriumaluminiumsilikate, insbesondere Zeolith A, organische Polymere, insbesondere Polymethacrylate oder Gemische dieser Reibkörper,vorzugsweise in Mengen von 1 bis 30Gew.%, vorzugsweise von 2,5 bis 25 Gew.% und insbesondere von 5 bis 20 Gew.%, jeweils bezogen auf das gesamte Mittel, enthalten.

Als Träger für die Zahnpasten, der die Einstellung einer geeigneten Konsistenz für die Dosierung aus Tuben, Spendebehältern oder flexiblen Flaschen ermöglicht, eignet sich beispielsweise eine Kombination aus Feuchthaltemitteln, Bindemitteln und Wasser.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den Zahnpasten zur Unterstützung der Reinigungswirkung und gewünschtenfalls auch zur Entwicklung von Schaum beim Zähnebürsten sowie zur Stabilisierung der Polierkörperdispersion im Träger in einer Menge von 0,1―5 Gew.-% enthalten.

Geeignete Tenside sind z. B. lineare Natriumalkylsulfate mit 12―18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12―16 C-Atomen in der linearen Alkylgruppe und 2―6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8―18 C-Atomen in der Acylgruppe. Auch zwitterionische, ampholytische und nichtionische Tenside sind geeignet, z. B. Oxethylate von Fettsäuremono- und - diglyceriden, von Fettsäure-Sorbitanestem und Alkyl(oligo)-Glucoside.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 ― 0,3 Gew.-% eingesetzt.

D-Panthenol D ― (+) ― 2,4-Dihydroxy-N-(3-hydroxypropyl)-3,3-dimethyl-butyramid zeigt eine der Pantothensäure entsprechende biologische Aktivität. Die Pantothensäure (R ― (+) ―N―(2,4-Dihydroxy-3,3-dimethylbutyryl-ß-alanin) ist eine Vorstufe in der Biosynthese des Coenzyms A und wird zum Vitamin-B-Komplex (B3) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 ― 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 ― 22 C-Atomen verwendet. Besonders bevorzugt ist Retinol-Palmitat geeignet. Bei Verwendung eines Retinol-Esters, z.B. Retinol-Palmitat mit einer Aktivität von 1,7 · 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.
Bevorzugte Zahnpflegemittel gemäß der vorliegenden Erfindung enthalten neben Poliermitteln, Fluorverbindungen, Feuchthaltemitteln und Bindemitteln bevorzugt

| | | |
|---|---|---|
| 0,01 - 1 | Gew.-% | eines halogenierten Diphenylethers |
| 0,05 - 5 | Gew.-% | Panthenol oder ein Salz der Pantothensäure und |
| 0,01 - 0,1 | Gew.-% | eines Retinol-Esters, bevorzugt Retinol-Palmitat. |

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure ― oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 ― 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten sind
- Oberflächenaktive Stoffe, bevorzugt anionische, zwitterionische, amphotere, nicht- ionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronen- säure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Ein weiterer bevorzugter Inhaltsstoff, der in den erfindungsgemäßen Mitteln enthalten sein kann, ist das N-Acetyl-L-cystein (NAC). Erfindungsgemäße Mittel, die zusätzlich NAC enthalten, zeichnen sich durch besonders positive entzündungshemmende und zahnfleischreparierende Eigenschaften aus. Hier sind erfindungsgemäße Mund- und Zahnpflege- und ―reinigungsmittel bevorzugt, die zusätzlich N-Acetyl-L-cystein (NAC), vorzugsweise in Mengen von 0,0001 bis 5 Gew.%, vorzugsweise von 0,001 bis 2,5 Gew.% und insbesondere von 0,01 bis 1 Gew.%, jeweils bezogen auf das gesamte Mittel, enthalten.

Weitere bevorzugte Inhaltsstoffe, die in den erfindungsgemäßen Mitteln enthalten sein können, sind Thymulin- und/oder Thymopietinpeptide. Unter diesen sind die Petide bevorzugt, welche mindestens eine der folgende Aminosäuresequenzen aufweisen:
Gln-Gly-Gly
Arg-Lys-Asp
Lys-Asp-Val

Bevorzugte Aminosäuresequenzen der Peptide lassen sich durch die allgemeine Formel
X-Gln-Gly-Gly-Y

Beschreiben, in der Gln für Glutaminsäure oder ein Glutaminsäurederivat und Gly für Glycin oder ein Glycinderivat steht. Besonders bevorzugte Peptide besitzen eine Sequenz der Formel
A-X-Gln-Gly-Gly-Y,

In der A für einen Mono- oder Dicarbonäsurerest, vorzugsweise einen Acetylrest, steht und
-X für Ser, Lys-Ser, Ala-Lys-Ser, Pyr-Ala-Lys-Ser, eine chemische Bindung oder Glx-Ala-Lys-Ser steht, wobei Glx für Pyro-Glu, Glu oder Gly und ihre jeweiligen Derivate steht M;
-Y für Ser-Asn-OH, Ser-Asn-NH₂, Ser-OH oder Ser-NH₂ steht.

Bevorzugte Peptide weisen die folgenden Aminosäuresequenzen auf:
A-Pyr-Ala-Lys-Ser-Gln-Gly-Gly-Ser-Asn-NH₂
A-Pyr-Ala-Lys-Ser-Gln-Gly-Gly-Ser-NH₂
A-Ala-Lys-Ser-Gln- Gln-Gly-Ser-Asn-NH₂
A-Ala-Lys-Ser-Gln-Gly-Gly-Ser-NH₂
A-Lys-Ser-Gln-Gly-Gly-Ser-Asn-NH2
A-Lys-Ser-Gln-Gly-Gly-Ser-NH2
A-Ser-Gln-Gly-Gly-Ser-Asn-NH₂
A-Ser-Gln-Gly-Gly-Ser-NH₂
A-Gln-Gly- Gly-Ser-Asn-NH₂
A-Gln-Gly-Gly-Ser--NH₂
A-Ala-Lys-Ser-Gln-Gly-Ser-Asn-OH

Andere bevorzugte Peptide besitzen eine Sequenz der Formel
A-W-Lys-Asp-Z,

In der A für einen Mono- oder Dicarbonäsurerest, vorzugsweise einen Acetylrest, steht und
-W für Glu-Gln-Arg, Gln-Arg, Arg, Arg-Lys, Arg-Lys-Asp oder eine chemische Bindung steht;
-Z für Val-Tyr-NH₂, Val-Tyr-OH, Val-NH₂, Val-OH, Tyr-OH, Tyr-NH₂, -OH oder-NH₂ steht.

Bevorzugte Peptide weisen die folgenden Aminosäuresequenzen auf:
A-Glu-Gln-Arg-Lys-Asp-Val-Tyr-NH₂
A-Glu-Gln-Arg-Lys-Asp-Val-Tyr-OH
A-Gln-Arg-Lys-Asp-Val-Tyr-NH₂
A-Gln-Arg-Lys-Asp-Val-Tyr-OH
A-Arg-Lys-Asp-Val-Tyr-NH₂
A-Arg-Lys-Asp-Val-Tyr-OH
A-Lys-Asp-Val-Tyr-NH₂
A-Lys-Asp-Val-Tyr-OH
A-Arg-Lys-Asp-Val-NH₂
A-Arg-Lys-Asp-Val-OH
A-Arg-Lys-Asp-NH₂
A-Arg-Lys-Asp-OH

Zusammenfassend sind erfindungsgemäße Mund- und Zahnpflege- und ―reinigungsmittel bevorzugt, die zusätzlich Thymulin- und/oder Thymopietinpeptide, vorzugsweise mit mindestens drei Aminosäuren, vorzugsweise in Mengen von 0,0000001 bis 1 Gew.%, vorzugsweise von 0,000001 bis 0,01 Gew.% und insbesondere von 0,000005 bis 0,0001 Gew.%, jeweils bezogen auf das gesamte Mittel, enthalten.

Die erfindungsgemäßen Mittel, die zusätzlich zum Protein aus Legminosensamen NAC und/oder Thymulin- und/oder Thymopietinpeptide enthalten, sind besonders bevorzugte Ausführungsformen der vorliegenden Erfindung.

Weitere Gegenstände der vorliegenden Erfindung sind die Verwendung von Proteinen aus Leguminosensamen in Mundpflegemitteln, Mundreinigungsmitteln, Zahnpflegemitteln oder Zahnreinigungsmittel, die Verwendung von Proteinen aus Leguminosensamen zur Herstellung eines Mittels zur Behandlung von entzündlichen Erkrankungen des Mundes und der Mundhöhle, insbesondere zur Behandlung der Gingivitis sowie die die Verwendung von Proteinen aus Leguminosensamen zur Herstellung eines Mittels zur Reparatur geschädigten Zahnfleisches. Bezüglich bevorzugter Einsatzkonzentrationen, bevorzugter weiterer Inhaltsstoffe der jeweiligen Mittel und weitere bevorzugter Ausführungsformen gilt mutatis mutandis das zu den erfindungsgemäßen Mitteln Gesagte.

## Patentansprüche

1. Mund- und Zahnpflege- und -reinigungsmittel, enthaltend Feuchthaltemittel und mindestens ein Protein aus Leguminosensamen.

2. Mund- und Zahnpflege- und ―reinigungsmittel nach Anspruch 1, **dadurch gekennzeichnet, daß** es ein Protein enthält, das **gekennzeichnet ist durch**
- a) seine Isolierung aus Leguminosensamen,
- b) mindestens eine Bande in der Polyacrylamidgelelektrophorese mit Natriumdodecylsulfat,
- c) Molekularmassen von 3000 bis 30'000 g/mol,
- d) Gehalt an Gesamtstickstoff von 14% bis 20% und Aminostickstoff von 1 % bis 2% bezogen auf den Proteingehalt,
- e) Löslichkeit in Wasser und wässrigen Elektrolytlösungen und Unlöslichkeit in Ethanol und Aceton,
- f) starke Ausfällung in wässriger Lösung nach Zusatz von Trichloressigsäure, Sulfosalicylsäure, Pikrinsäure oder Benzethoniumchlorid,
- g) Hemmung von PMN-Elastase und Fibroblastenelastase.

3. Mund- und Zahnpflege- und -reinigungsmittel nach einem der Ansprüche 1 oder 2, **dadurch gekennzeichnet, daß** es mindestens ein Protein aus Sojasamen enthält.

4. Mund- und Zahnpflege- und -reinigungsmittel nach einem der Ansprüche 1 bis 3, **dadurch gekennzeichnet, daß** es das/die Protein(e) aus Leguminosensamen in Mengen von 0,0001 bis 5 Gew.%, vorzugsweise von 0,001 bis 2,5 Gew.% und insbesondere von 0,01 bis 1 Gew.%, jeweils bezogen auf das gesamte Mittel, enthält.

5. Mund- und Zahnpflege- und ―reinigungsmittel nach einem der Ansprüche 1 bis 4, **dadurch gekennzeichnet, daß** es ein Feuchthaltemittel aus der Gruppe der Alkohole mit mindestens 2 OH-Gruppen, vorzugsweise Glycerin, Sorbit, Mannit, Xylitol, Polyethylenglycol, Polypropylenglycol und deren Mischungen, enthält.

6. Mund- und Zahnpflege- und -reinigungsmittel nach einem der Ansprüche 1 bis 5, **dadurch gekennzeichnet, daß** es zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel, enthält.

7. Mund- und Zahnpflege- und ―reinigungsmittel nach einem der Ansprüche 1 bis 6, **dadurch gekennzeichnet, daß** es zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthält.

8. Mund- und Zahnpflege- und -reinigungsmittel nach einem der Ansprüche 1 bis 7, **dadurch gekennzeichnet, daß** es zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper,vorzugsweise in Mengen von 1 bis 30Gew.%, vorzugsweise von 2,5 bis 25 Gew.% und insbesondere von 5 bis 20 Gew.%, jeweils bezogen auf das gesamte Mittel, enthält.

9. Mund- und Zahnpflege- und ―reinigungsmittel nach einem der Ansprüche 1 bis 8, **dadurch gekennzeichnet, daß** es zusätzlich N-Acetyl-L-cystein (NAC), vorzugsweise in Mengen von 0,0001 bis 5 Gew.%, vorzugsweise von 0,001 bis 2,5 Gew.% und insbesondere von 0,01 bis 1 Gew.%, jeweils bezogen auf das gesamte Mittel, enthält.

10. Mund- und Zahnpflege- und ―reinigungsmittel nach einem der Ansprüche 1 bis 9, **dadurch gekennzeichnet, daß** es zusätzlich Thymulin- und/oder Thymopietinpeptide, vorzugsweise mit mindestens drei Aminosäuren, vorzugsweise in Mengen von 0,0000001 bis 1 Gew.%, vorzugsweise von 0,000001 bis 0,01 Gew.% und insbesondere von 0,000005 bis 0,0001 Gew.%, jeweils bezogen auf das gesamte Mittel, enthält.

11. Verwendung von Proteinen aus Leguminosensamen in Mundpflegemitteln, Mundreinigungsmitteln, Zahnpflegemitteln oder Zahnreinigungsmitteln.

12. Verwendung von Proteinen aus Leguminosensamen zur Herstellung eines Mittels zur Behandlung von entzündlichen Erkrankungen des Mundes und der Mundhöhle, insbesondere zur Behandlung der Gingivitis.

13. Verwendung von Proteinen aus Leguminosensamen zur Herstellung eines Mittels zur Reparatur geschädigten Zahnfleisches.
